# EUROPEAN PATENT SPECIFICATION

(11) **EP 3 389 528 B1**
(45) Date of publication and mention of the grant of the patent: **18.09.2019**
(21) Application number: 16810395.0
(22) Date of filing: 15.12.2016
(51) Int. Cl.: A61B 18/14, A61F 9/007

(54) **MICROSURGICAL FINE GRIPPING AND DIATHERMY FORCEPS AND DIATHERMY CUTTING DEVICE FOR INTRAOCULAR SURGERY**
MIKROCHIRURGISCHE, FEIN GREIFENDE UND DIATHERMIE-ZANGE UND -SCHERE
PRÉHENSION FINE DE MICROCHIRURGIE, FORCEPS DIATHERMIQUES ET CISEAUX

(30) Priority: 15.12.2015 EP 15200265
(43) Date of publication of application: 24.10.2018
(73) Proprietor: Netvlieschirurg B.V., 3084 LS Rotterdam (NL)
(72) Inventor: VAN OVERDAM, Koen-Willem Adriaan, 3084 LS Rotterdam (NL)
(74) Representative: Nederlandsch Octrooibureau
(86) International application number: PCT/EP2016/081245
(87) International publication number: WO 2017/102975

(56) References cited:
- EP-A2- 1 325 710
- WO-A1-2014/172503
- US-A- 5 207 691
- US-A- 5 342 381
- US-A- 5 352 222
- US-A- 5 697 949
- US-A- 5 984 939
- US-A1- 2014 046 325
- US-A1- 2014 172 010
- US-A1- 2015 105 820

## Description

### FIELD OF THE INVENTION

The invention relates to a microsurgical fine gripping and diathermy forceps for intraocular surgery. The invention further relates to a membrane forceps for performing a vitreoretinal membrane peel procedure. The invention further relates to a microsurgical cutting and diathermy device.

### BACKGROUND OF THE INVENTION

During ophthalmologic microsurgical procedures conducted to treat, for example, complicated diseases of the retina, it is known to use a pair of tweezers to remove tissue. This procedure can sometimes cause a hemorrhage as a side effect. To stop this bleeding, a diathermy device is known, which has sharp-pointed coaxial bipolar diathermy tip. Such a device can be used to close a blood vessel by cauterization. Using the tip, a blood vessel can be compressed from one side. The exchange of the tweezers for a diathermy instrument takes time and increases the complexity of the procedure. During the exchange of devices inserted in the human body, in particular the eye, the bleeding can increase.

A bipolar diathermy forceps is an instrument known in macro-surgery to seal blood vessels to prevent bleeding during and after surgery. A diathermy in the form of a pair of tweezers allows a blood vessel to be grasped and closed by being pinched, while cauterizing the blood vessel.

US5868728 discloses an intraocular forceps that can be inserted through a retinotomy to perform a choroidal biopsy; the members (which can be two or more in number) may also be rotatably connected to a plunger, and means for rotating members in order to ensure a cleaner incision, or a better cutting and tearing action. An intraocular cauterization device (e.g. electrocauterization probe) can be included to reduce bleeding following the excision of tissue to biopsied and its retraction within a cannula.

US2014/0172010 A1 discloses a membrane forceps for performing a retinal membrane peeling procedure. This document discloses the preamble of claim 1.

EP 1 325 710 A2 discloses a microsurgical pair of scissors for intraocular surgery. This document discloses the preamble of claim 14.

### SUMMARY OF THE INVENTION

It is an object of the invention to provide an improved microsurgical fine gripping forceps.

An object is to provide a device that makes it possible to use a bipolar diathermy tweezers during microsurgical procedures, and combines the function of a micro-forceps for removal of tissue with the function of a diathermy for cauterization of blood vessels, by creating separate positive pole and negative pole.

Another object is to provide a microsurgical diathermy having the shape of forceps, making it more effective to coagulate blood vessels, and thereby preventing bleeding in microsurgical procedures, thereby not having to switch between forceps and diathermy device during microsurgical procedures.

Another object is to provide a microsurgical diathermy forceps, which is easy to manufacture.

An aspect of the invention is a microsurgical fine gripping and diathermy forceps for intraocular surgery, comprising:
a tube defining a conduit and having an outer diameter of at most 1 millimeter; and
forceps jaws protruding from the conduit at a distal end of the tube, the forceps jaws comprising at least a first forceps jaw and a second forceps jaw, wherein the forceps jaws are configured such as to allow grasping a vitreoretinal membrane;
wherein at least two of the first forceps jaw, the second forceps jaw, and the tube are configured such as to form bipolar electrodes for providing a bipolar diathermy at the forceps jaws and/or a distal end of the tube.

The microsurgical device combines a fine gripping forceps with a diathermy. This improves the ability of the device to cauterize tissues, because the forceps allows to cauterize tissues with greater precision and/or to cauterize tissues better. Moreover, the combination improves the efficiency by preventing the need to exchange a forceps for a cauterization probe when a hemorrhage occurs during a tissue removal procedure, such as a membrane peeling procedure.

The forceps may comprise an elongated element which extends through the conduit of the tube and is movable with respect to the tube and comprises at least one electrically conducting core which extends through the conduit of the tube, wherein the forceps jaws are fixed to the elongated element, and wherein the at least one electrically conducting core is electrically connected to at least one of the forceps jaws. The elongated element allows a precise control and fixed alignment of the forceps jaws during the procedure.

The elongated element may comprise an electrically conducting first core, an electrically conducting second core, and an electrically isolating layer which electrically isolates the first core from the second core, wherein a first forceps jaw of the forceps jaws is electrically connected to the first core, and a second forceps jaw of the forceps jaws is electrically connected to the second core, wherein the first core, the second core, and the isolating layer extend from the forceps jaws through the tube towards a proximal end of the tube. This configuration allows the forceps jaws of a microsurgical fine gripping forceps to act as bipolar electrodes during a cauterization procedure.

The isolating layer may comprise an adhesive configured to fix the first core with respect to the second core. This provides an effective way to provide a single elongated element comprising two electrically isolated cores. The isolating layer with the adhesive may thus be configured to fix the first core and the second core to the isolating layer.

A cross section of the first core may have a shape of a circular segment and a cross section of the second core may have a shape of a circular segment, wherein a straight side of the cross section of the first core faces a straight side of the cross section of the second core. This way, an elongated element with a substantially circular cross section may be created. For example, the straight sides of the cross sections may match with respect to length.

The forceps may comprise a further electrically isolating layer configured to electrically isolate the first core and/or the second core from the tube. This avoids an electric shortcut between the two cores.

The tube may be electrically conducting, and the forceps may comprise a further electrically isolating layer configured to electrically isolate the at least one core of the elongated element from the tube. This allows the at least one core and the tube to provide separate electrically conducting paths for two bipolar cauterization electrodes.

The at least one core may be configured to be electrically connected to a first pole and the tube may be configured to be connected to a second pole of a source of electric energy. This allows the at least one core and the tube to provide separate electrically conducting paths between two bipolar cauterization electrodes and the poles of the source of electric energy.

The tube may be longitudinally displaceable with respect to the at least one core and the forceps jaws, so that the forceps jaws can be placed in a retracted position in which the forceps jaws are at least partially retracted inside the tube and an extended position in which the forceps jaws are extended further out of the tube than in the retracted position, and wherein the isolating layer is configured to electrically isolate the forceps jaws from the tube in the retracted position. This allows the forceps jaws and the tube to act as bipolar cauterization electrodes.

The forceps may comprise a further (i.e. inner) tube inside the (i.e. outer) tube, wherein the core extends through the inner tube, wherein the forceps jaws are configured to pinch together when the inner tube is moved in the distal direction and wherein the outer tube is movable in the distal direction so that the distal tip of the outer tube can extend beyond the distal tip of the inner tube. This allows the outer tube to reach out closely to the tip of the forceps jaws.

The further electrically isolating layer may be in between an inner surface of the tube and an outer surface of the further tube. This is a suitable way to provide the further electrically isolating layer. For example, it allows to provide a single electrically isolating layer that electrically isolates the outer tube from all of the inner tube, the core, and the forceps jaws.

The forceps may comprise a first control for controlling the movement of the further tube and a second control for controlling the movement of the tube. This allows to manipulate the tubes of the forceps.

The forceps may comprise a third control for controlling a switch to switch on and off an electric source. This allows to control application of cauterizing electricity.

According to another aspect, a microsurgical cutting and diathermy device for intraocular surgery is provided, comprising:
a tube defining a conduit and having an outer diameter of at most 1 millimeter; and
a pair of scissor blades protruding from the conduit at a distal end of the tube, the scissor blades comprising at least a first scissor blade and a second scissor blade;
wherein the scissor blades are configured such as to allow cutting a vitreoretinal membrane;
wherein at least two of the first scissor blade, the second scissor blade, and the tube are configured such as to form bipolar electrodes for providing a bipolar diathermy at the scissor blades and/or a distal end of the tube.

This allows microsurgery involving cuts in tissue to be efficiently combined with cauterization by means of the diathermy.

The device may further comprise an elongated element which extends through the conduit of the tube and is movable with respect to the tube and comprises at least one electrically conducting core which extends through the conduit of the tube, wherein the scissor blades are fixed to the elongated element, and wherein the at least one electrically conducting core is electrically connected to at least one of the scissor blades.

In a particular example, the tube may be electrically conducting, and wherein the device comprises a further electrically isolating layer configured to electrically isolate the at least one core from the tube.

The tube may be longitudinally displaceable with respect to the at least one core and the scissor blades, so that the scissor blades can be placed in a retracted position in which the scissor blades are at least partially retracted inside the tube and an extended position in which the scissor blades are extended further out of the tube than in the retracted position, and wherein the isolating layer is configured to electrically isolate the scissor blades from the tube in the retracted position.

The microsurgical cutting and diathermy device may comprise a further tube inside the tube, wherein the core extends through the further tube, wherein the scissor blades are configured to pinch together when the further tube is moved in the distal direction and wherein the tube is movable in the distal direction so that the distal tip of the tube can extend beyond the distal tip of the further tube.

The person skilled in the art will understand that the features described above may be combined in any way deemed useful. Features described in respect of the microsurgical fine gripping and diathermy forceps may be applied in a similar way to the microsurgical cutting and diathermy device, and vice versa.

### BRIEF DESCRIPTION OF THE DRAWINGS

In the following, aspects of the invention will be elucidated by means of examples, with reference to the drawings. The drawings are diagrammatic and may not be drawn to scale. Throughout the drawings, similar items may be labeled with the same reference numerals.
Fig. 1 shows a first example of a fine gripping diathermy forceps.
Fig. 2 shows components of the fine gripping diathermy forceps.
Fig. 3A, 3B, 3C, and 3D show different forceps jaws configurations.
Fig. 4 shows a partially worked open example of a tube of the fine gripping diathermy forceps.
Fig. 5 shows a proximal part of the fine gripping diathermy forceps.
Fig. 6A shows a cross section of a second example of a fine gripping diathermy forceps.
Fig. 6B shows a top view of the second example of a fine gripping diathermy forceps.
Fig. 6C shows a side view of the second example of a fine gripping diathermy forceps.
Fig. 7 shows a third example of a fine gripping diathermy forceps.
Fig. 8 shows a worked open view of a distal portion of the second and third example of a fine gripping diathermy forceps.
Fig. 9A shows a distal portion of the second and third example of a fine gripping diathermy forceps with the jaws in an open position.
Fig. 9B shows a distal portion of the second and third example of a fine gripping diathermy forceps with the jaws in a close position.
Fig. 10A shows a distal portion of a fourth example of a fine gripping diathermy forceps with the jaws in an open position.
Fig. 10B shows a distal portion of the fourth example of a fine gripping diathermy forceps with the jaws in a close position.
Fig. 11 shows a proximal portion of the fourth example of a fine gripping diathermy forceps.
Fig. 12 shows a distal portion of a configuration, in which the forceps jaws have been replaced with scissor blades.
Fig. 13A shows the scissor blades in an open position.
Fig. 13B shows the scissor blades in a close position.

### DETAILED DESCRIPTION OF EMBODIMENTS

The following description with reference to the accompanying drawings is provided to assist in a comprehensive understanding of various embodiments of the present disclosure as defined by the claims and their equivalents. It includes various specific details to assist in that understanding, but these are to be regarded as merely exemplary. Accordingly, those of ordinary skill in the art will recognize that various changes and modifications of the various embodiments described herein can be made without departing from the scope and spirit of the present disclosure. In addition, descriptions of well-known functions and constructions may be omitted for clarity and conciseness.

A fine gripping forceps can be used for removing e.g. membranes on the retina (known as epiretinal membranes, ERMs) and/or the inner layer of the retina, in particular the internal limiting membrane (ILM). Such procedures may be necessary in treatment of e.g. macular pucker, macular hole, as well as more complex diseases such as Proliferative Diabetic Retinopathy, and/or Proliferative Vitreoretinopathy.

In case of a hemorrhage during a procedure, a diathermy may be necessary to close a blood vessel.

For example, a combination forceps, which can be used both for removing the relevant tissue and for closing blood vessels, would be a desirable device. For example, treatment of tumors of the retina, such as retinal angiomas or hemangioblastomas. These angiomas are often associated with fractional ERMs, which can be removed successfully using fine gripping forceps, but complete closure of the typical thickened feeder and drainer vessels of the lesion is difficult using the existing diathermy, causing hemorrhage during or after excision of the lesion.

Using the diathermy forceps according to the present disclosure, the blood vessel can be pressed shut by pinching the vessel in between the forceps jaws, and can subsequently be cauterized. The fine gripping and diathermy forceps can also be useful in other applications and/or diseases where both a forceps and/or a diathermy are regularly used.

In certain embodiments, a forceps can be combined with a diathermy for microsurgery (such as intraocular and/or vitreoretinal surgery).

In certain embodiments, a diathermy function may be implemented by replacing a single forceps core by two equal parts, separated longitudinally by electrically isolated material. Each part of the core is electrically and mechanically connected to another forceps jaw. The parts of the core may also be electrically isolated from the surrounding tube. This way, each part of the core (including the respective attached forceps jaw) forms an electric pole, so that the two poles can be charged with respect to each other using an electric radio-frequency (RF) signal generator to cauterize the tissue in between the two forceps jaws.

The two parts of the core may be isolated from each other by means of an electrically isolating adhesive. A suitable adhesive material is Araldite 2014-1. Alternatively a composition comprising such an adhesive material together with another material, preferably another electrically isolating material, may be used. For example, a mixture of Araldite 2014-1 and polyimide may be used. In another example, a multi-layer structure may be used, wherein at least one layer is electrically isolating and at last one layer comprises an adhesive.

Araldite 2014-1, which denotes a two component epoxy paste adhesive, is a trademark of Huntsman Advanced Materials. This material is merely mentioned herein as an example of an adhesive. Other adhesive materials, such as epoxy resins or other adhesives, may be used instead.

Polyimide is a polymer thermoset plastic that has thermal stability, chemical resistance, and tensile strength. These characteristics make polyimide suitable for, inter alia, surgical and medical tubing applications. However, other plastics or other electrically isolating materials may be used instead of polyimide.

For example, a method of creating two forceps core portions may comprise splitting an existing forceps core longitudinally. This splitting can be performed using a laser cutter device, for example. Alternatively, the core portions may be manufactured separately.

According to another example, a diathermy function is implemented by electrically isolating the forceps core (which may be a single core to which both the forceps jaws are electrically and mechanically connected) from the surrounding tube. This creates two electric poles: one pole formed by the core and the forceps jaws, and one pole formed by the tube. This isolation may be performed by for example a tube made of polyimide around the core but inside the tube. For example, the isolation may be adhesive on one side, so that it attaches to either one of the inside wall of the tube or the core, allowing movement of the core with respect to the tube.

According to another example, an inner tube is provided for operating the position of the forceps jaws, to control a pinching action thereof by moving the tube longitudinally. An outer tube is provided to perform a diathermy function. The inner tube may be disposed inside a conduit defined by outer tube. The elongated element may be disposed inside the inner tube. The outer tube may be configured to be movable longitudinally so that the tip of the outer tube reaches out further towards the tip of the forceps jaws than the inner tube can. For example, the conduit defined by the outer tube may be large enough so that the forceps jaws (or scissor blades, as the case may be) may be retracted beyond the widest point of the forceps jaws and/or scissor blades, when they are in the close position.

Independent of the formation of the electric poles, the shape of the forceps jaws may be selected in several different ways, depending on the application. Different forceps jaws may be designed, for example, for peeling of ERM and/or ILM, and for gripping of thickened blood vessels.

The forceps may have one of a plurality of different sizes. For example, the tube size may be in the range of, for example, 0.2 mm to 1 mm. Suitable examples of tube sizes are: 0.9 mm, equivalent to 20-Gauge; 0.6 mm, which is equivalent to 23-Gauge, 0.5 mm, which is equivalent to 25-Gauge, and 0.4 mm, which is equivalent to 27-Gauge. These dimensions relate to the outside diameter of the tube.

Also, different kinds of material may be used to create the forceps. Metals are particularly advantageous. For the electric poles, an electrically conducting material should be used.

At the proximal end, the forceps has terminals to connect a diathermy apparatus. The diathermy apparatus is configured to generate an electric signal, at the terminals which causes electrons to pass through the tissue near the tip of the forceps jaws and/or the tip of the tube, so that the tissue becomes hot and cauterizes. Different brands and types of diathermy apparatus can be connected to the terminals, if necessary by designing the terminals appropriately, according to specifications of the diathermy apparatus. Alternatively, the diathermy apparatus may be integrated in the forceps.

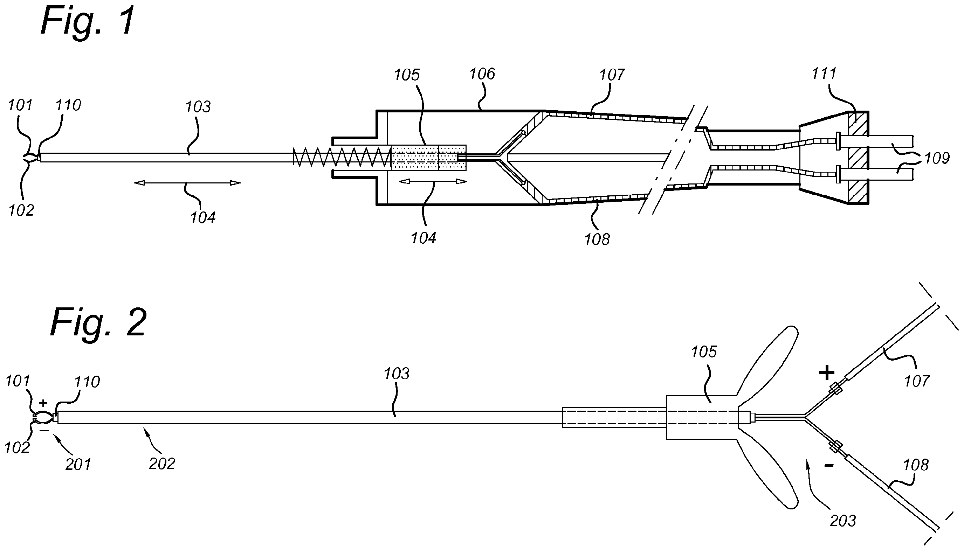

Fig. 1 to 5 illustrate a first example of a microsurgical bipolar diathermy forceps. The forceps comprises a pair of forceps jaws 101, 102 protruding from a tube 103. The forceps jaws 101, 102 are connected to a first core 401 and a second core 402 of an elongated element 403, which extends from a basis of a forceps jaw near the distal end of the tube through the tube towards the proximal end of the tube. Beyond the proximal end of the tube, the first core 401 is connected to electric wire 107, and the second core 402 is connected to electric wire 108. These wires connect the cores to respective electric terminals 109 of a connector 111. The forceps also comprises a handle 106, which can be used to hold the forceps. Also, the handle 106 may comprise a control (not shown) to allow the user to control movement of the tube 103 in the longitudinal direction with respect to the forceps jaws 101, 102 and the elongated element 403 with the first core 401 and second core 402, as indicated by double arrow 104. To that end, the tube 103 may be fixed to a block 105, which may be made of a plastic, for example, and the block 105 can also move in the same direction, as indicated by arrows 104. The movement of the plastic block 105 may be controlled by a control, such as a button or switch, provided on the handle 106. For example, the plastic block 105 may be made of a flexible plastic. When the tube 103 is moved in the distal direction, the tube 103 partially absorbs the forceps jaws 101, 102, and the latter are then forced together in a pinching action, due to the narrow passage within the tube 103.

The handle 106 may have a control (not shown) configured to control the movement of the tube with respect to the forceps jaws, and thus vary the forceps jaws between at least a closed position in which the forceps jaws meet to exert a pinching force, and an open position in which the forceps jaws are apart from each other, and wherein the forceps is configured to apply an electric current to the electric terminals with the forceps jaws in the closed position.

The electric wires 107, 108 may be glued to the handle 106 or otherwise disposed therein. The terminals 109 form an interface to a diathermy apparatus, which provides the electricity signals to the bipolar terminals. Such diathermy apparatus is known in the art by itself.

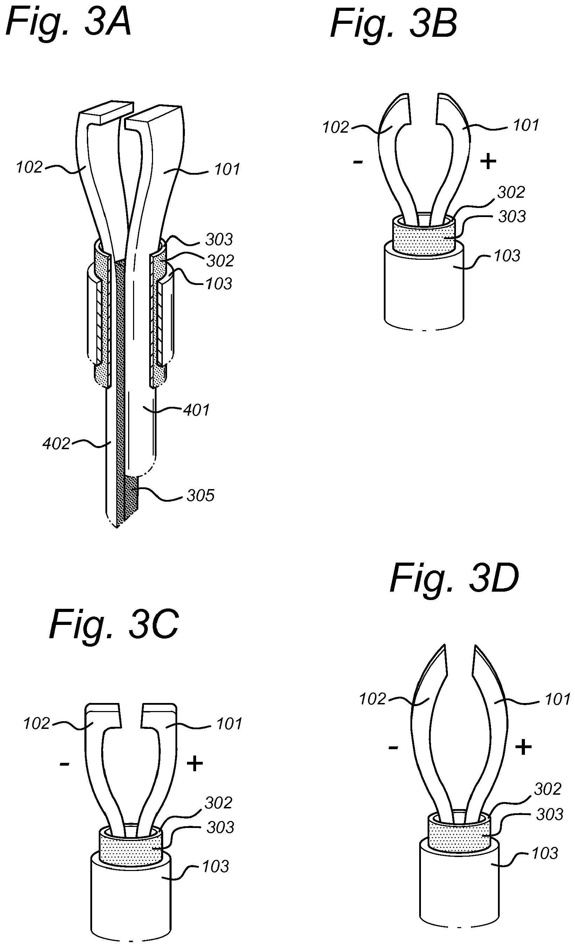

Fig. 2 shows the forceps of Fig. 1, however the handle 106 has not been drawn in Fig. 2. Fig. 3A shows a partially worked open perspective view of the portion 201 of the forceps of Fig. 2 containing the forceps jaws. Fig. 3B, 3C, and 3D show alternative examples of shapes of forceps jaws, which may be used in appropriate microsurgical procedures, such as vitreoretinal procedures. These jaws may replace the jaws shown in the example of Fig. 1 and 2. The types of jaws illustrated in Fig. 3A, 3B, 3C, and 3D may be used in combination with any of the forceps disclosed herein. Other types of jaws may also be used.

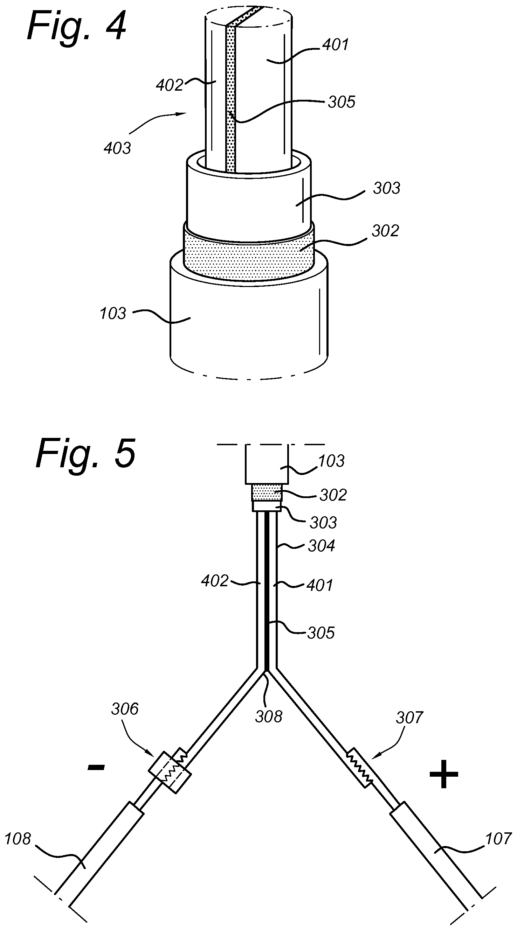

Fig. 4 shows a partially worked open portion 202 of the tube 103 with the cores 401, 402 disposed therein. Fig. 5 shows the portion 203 around the proximal end of the tube 103, in which the cores 401, 402 are connected to the electric wires 107, 108, respectively. For illustration purpose, two different kinds of connection are shown in Fig. 5 to connect the core to the electric wire: connection 306 is an adapter sleeve, whereas connection 307 is a soldered or heat-sealed joint. Either of these types of connections, or another type of connection, or a combination thereof, may be used. As shown in Fig. 3A, 4, and 5, the layers within the tube 103, from outside to inside, may include a tubular layer of an adhesive 302, such as Araldite 2014, with therein a tubular layer of an electrically insulating material 303, for example a plastic such as a polyimide tube. Using the adhesive, layer 302, the electrically insulating layer 303 may be fixed to the inside wall of the tube 103. Alternatively, the electrically insulating layer 303 may have a sufficient adhesive property by itself. Inside the tubular layer of electrically insulating material 303, the first core 401 and the second core 402 are disposed. The first core 401 and the second core 402 are separated by an electrically isolating layer 305. Preferably, this layer 305 is also adhesive so that it keeps the two cores 401 and 402 together. This layer 305 may comprise, for example, Araldite 2014. Alternatively, the layer 305 may comprise three layers: an electrically insulating layer with on both sides an adhesive layer. As shown in Fig. 3A, the forceps jaw 101 and the first core 401 are made of a single piece of material, in this case a metal. The forceps jaw 101 and the first core 401 may be cast as a whole. Alternatively, they may be joined by means of soldering or heat-sealing, for example. The same applies to the second forceps jaw 102 and the second core 402. The first core 401, the second core 402, the jaws 101, 102, and the tube 103 may be made of medical grade steel or another suitable material.

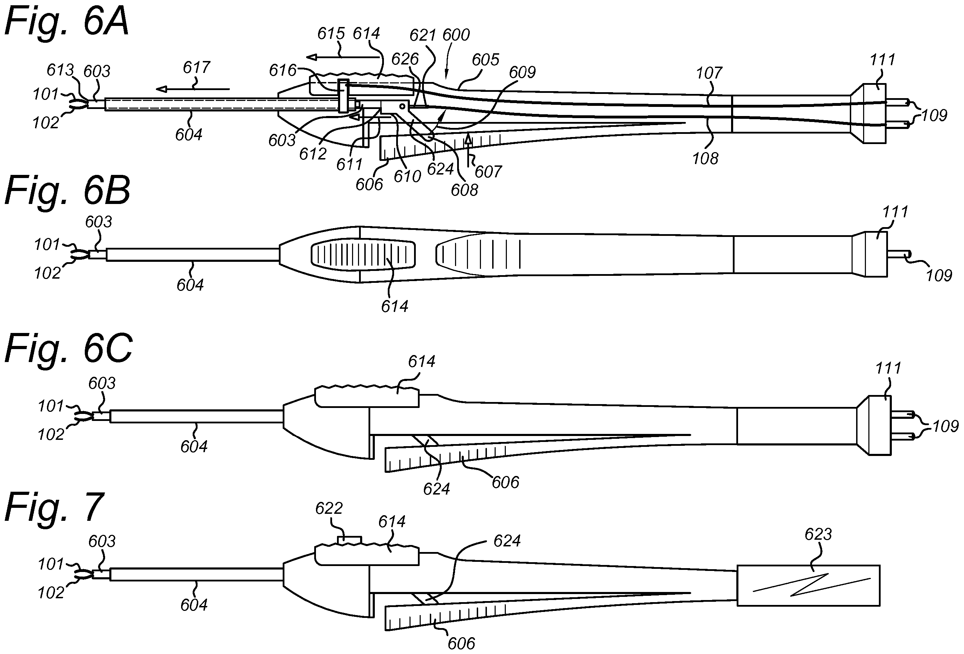

Fig. 6 shows several views of another example of a microsurgical fine gripping diathermy forceps. More specifically, Fig. 6A shows a cross sectional side view, Fig. 6B shows a top view, and Fig. 6C shows a side view. The connector 111 with connector terminals 109, and the electric wires 107, 108 are similar to those of Fig. 1. Also the forceps jaws 101 and 102 may be similar in shape to those of Fig. 1 and Fig. 3A, 3B, 3C, and/or 3D. In this example, the elongated element 626 comprises a single electrically conducting core 621, although this is not a limitation. The forceps jaws 101 and 102 are connected to the core 621. In certain embodiments, only one of the forceps jaws 101 or 102 may be connected to the core 621. The core 621 extends from at least one of the forceps jaws 101 and 102 to beyond the proximal end 612 of tube 603. The core 621 is connected via electric wire 108 to one of the terminals 109 of connector 111. The device comprises an inner tube 603 and an outer tube 604. At least part of the inner tube 603 is located inside the outer tube 604. At least part of the core 603 is located inside the inner tube 603. The forceps jaws 101, 102 protrude from the inner tube 603.

The handle 605 comprises a first control 606. When actuated by a user, the first control 606 causes the inner tube to move in a longitudinal direction (see arrow 617). In the example, the movement of the first control 606 in direction of arrow 607 causes a first end 608 of a pivoting body 624 to move approximately in the direction of arrow 609, and a second end 610 of the pivoting body 624 to move approximately in the direction of arrow 611. The second end 610 is mechanically coupled to the inner tube 603, so that the movement of second end 610 is transferred to the inner tube 603. However, it will be understood that the control of the movement of the inner tube 603 may be implemented in alternative ways.

The handle 605 also comprises a second control 614. When actuated by a user, the second control causes a longitudinal movement of the outer tube 604 in the direction of the arrow 617. In the example, the movement 614 is transferred to the outer tube 604 by means of a pin that is mechanically coupled to both the control 614 and the outer tube 604. However, the control of the movement of the outer tube 604 may be implemented in alternative ways.

The outer tube 604 may form a first electrode and at least one of the forceps jaws 101, 102, together with the core 621, may form a second electrode. In general, the inner tube 603 may be either part of the first electrode or part of the second electrode, or may be not part of either electrode. Appropriate electric isolation layer(s) may be provided to electrically separate the electrodes. In the example described herein, the inner tube 603 is part of the second electrode, together with the forceps jaws 101, 102 and the core 621.

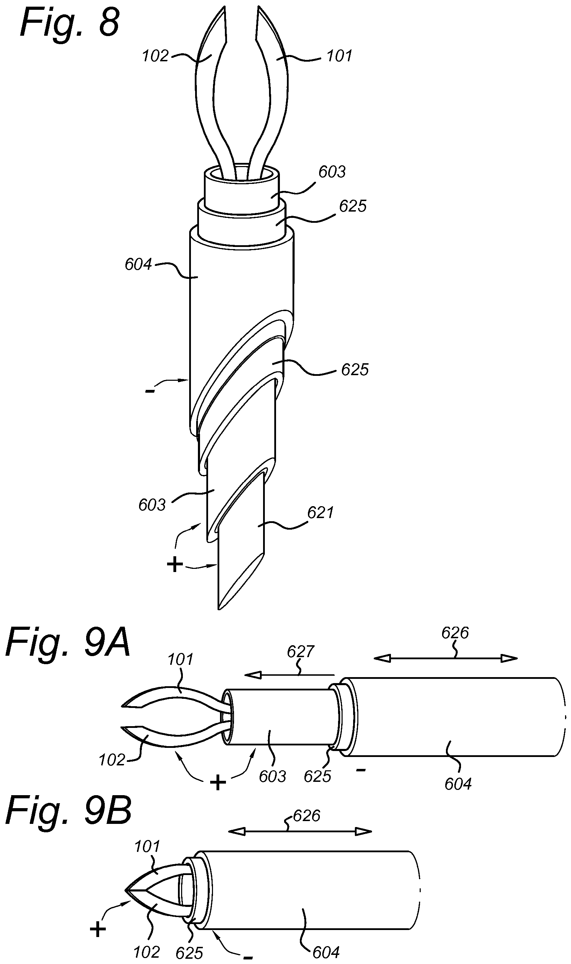

Fig. 8 shows a partially worked open enlarged view of the forceps jaws and the distal end of the tubes. From outside to inside, the outer tube 604, the electrically isolating layer (a tubular shaped layer) 625, the inner tube 603, and the core 621 connected to the forceps jaws 101, 102, are shown.

Fig. 9A and 9B show the forceps in two of the positions that may occur during use of the forceps, and which may be achieved using the controls 606, 614.

In the situation shown in Fig. 9A, the inner tube 603 protrudes from the outer tube 604. The forceps jaws 101, 102 protrude from the inner tube 603. The forceps jaws 101, 102 are biased outwardly, so that they are apart from each other. By operating the first control 606, the inner tube 603 can be moved longitudinally, as indicated by arrows 627. Due to the dimensions of the forceps jaws 101, 102 and the inner tube 603, the movement of the inner tube 603 in a distal direction causes the forceps jaws 101, 102 to close in a pinching action. When an object to be cauterized has been gripped in this manner, the outer tube 604 can be moved in a distal direction, so that the outer tube 604 covers the distal tip of the inner tube 603. This situation is illustrated in Fig. 9B. The electrically isolating layer 625 moves along with the outer tube 604. For example, the electrically isolating layer 625 is fixed to the inside surface of the outside tube 604 using an adhesive. Also, as illustrated, the electrically isolating layer 625 may protrude somewhat from the outer tube 604. Due to its larger inner diameter compared to the inner diameter of the inner tube and the axial dimensions of the forceps jaws, the distal tip of the outside tube 604 may be brought close to the distal tip of the forceps jaws 101, 102 without increasing the pinching force exerted by the forceps jaws to a gripped object. In this position, an electric potential or electric signal may be applied to the terminals 109 of the connector 111, which electric potential or signal is transferred to the forceps jaws 101, 102 and the distal tip of the outer tube 604. Here, the cauterization effect is exerted on the tissue.

After the cauterization is completed, the outer tube 604 may be retracted into the position shown in Fig. 9A using the second control 614, and the use of the forceps may be continued by movement of the inner tube 603 using the first control 606.

Fig. 7 shows another example in which the diathermy apparatus is integrated in the forceps. A third control 622 is provided to enable the electric potential or electric signal. A battery 623 or another type of electricity storage element or power supply is provided to provide electricity. Optional electric components (not shown) that convert the electric potential of the battery into a suitable cauterization signal may also be integrated. Similar integration of the diathermy apparatus in the forceps can be realized in the other example forceps disclosed herein (e.g. the ones shown in Figs. 1 through 6 and Figs. 10 and 11). It is noted that, although not shown in the drawings, such a third control 622 may also be provided, even if the cauterization apparatus and the electric source (e.g. battery) are not integrated in the forceps. For example, the third control can control an electric switch somewhere along the electric wires 107 and 108. Alternatively, a third terminal may be provided on connector 111, and a signal may be sent to the cauterization apparatus through that third terminal when the third control is activated, for example.

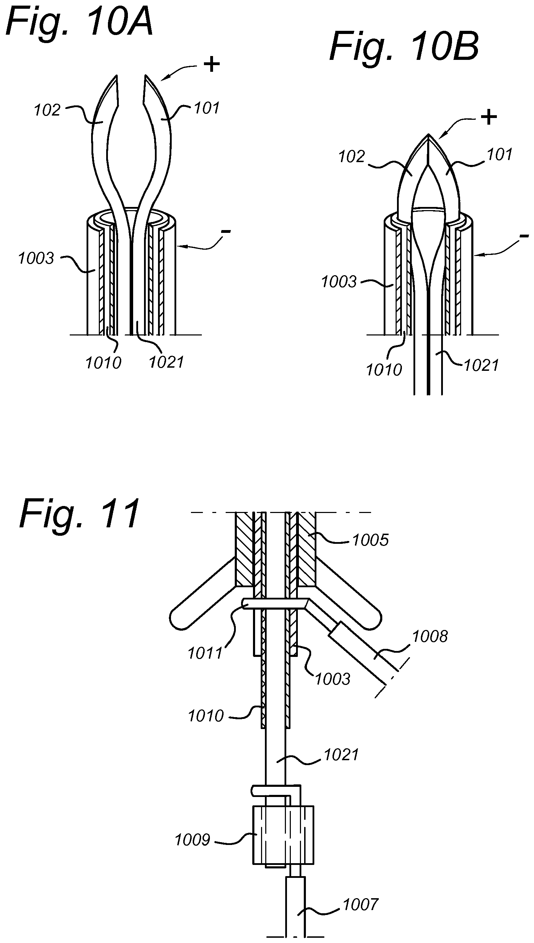

Fig. 10 and 11 show another example of a microsurgical fine gripping diathermy forceps. The working principle is comparable to the example of Fig. 6. However, a single tube 1003 is used to combine the function of the inner tube 603 and the outer tube 604 of Fig. 6. A tubular layer 1010 of electrically insulating material is used to electrically isolate the tube 1010 from the core 1021 and from the forceps jaws 101, 102. At the proximal end, the tube 1003 is connected to electric wire 1008 by means of e.g. a soldered connection or another suitable electric connection 1011. Also, the core 1021 is connected to electric wire 1007 via an electric connection 1009. The electric wires 1007, 1008 may at their other end be connected to the diathermy apparatus. The tube 1003 may be operated by means of a suitable control (not shown) to move longitudinally, thus moving between the situations drawn in Fig. 10A and Fig. 10B. In the situation drawn in Fig. 10A, the forceps jaws are drawn apart due to a bias. In the situation drawn in Fig. 10B, the forceps jaws are forced together by the tube 1003 so that they perform a pinching action. In that situation, the electricity may be applied to cauterize a tissue near the distal tip of the tube 1003 and the forceps jaws 101, 102.

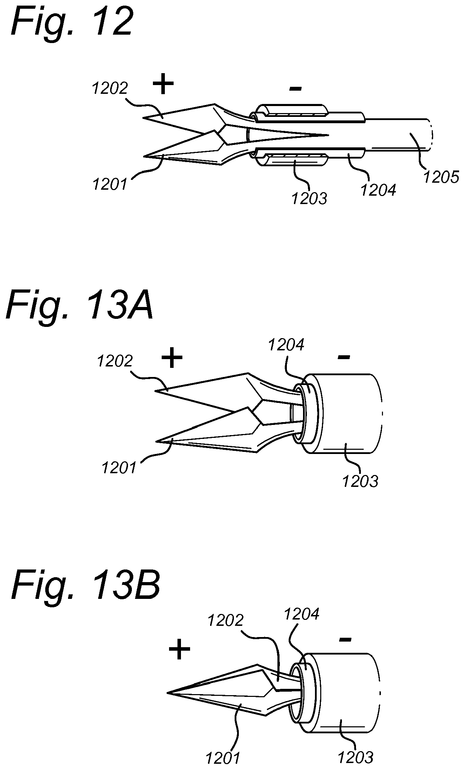

Fig. 12 shows a distal portion of a configuration, in which the forceps jaws have been replaced with scissor blades. It is observed that in all of the above described embodiments of the forceps, the forceps jaws may be replaced with scissor blades 1201, 1202 of a pair of scissors. The scissor blades 1201, 1202 are fixed to an elongated element 1205, which elongated element is movable with respect to a tube 1203. In certain embodiments, the tube 1203 is electrically conductive. The elongated element 1206 may be electrically isolated from the tube 1203 by means of an electrically isolating layer 1204. Fig. 12 shows the pair of scissors with an elongated element 1205 of a single electrically conductive core and with one electrically conductive tube 1203. This configuration is similar to the configuration of Fig. 10 and 11.

Although not shown in the drawing, the scissors can similarly be combined with the configuration with two tubes as described with reference to Figs. 6 to 9. In such a case, the inner tube 603 would be movable to perform the opening and closing action of the scissor blades 1201, 1202, and the outer tube 604 would slide further over the scissor blades 1201, 1202, so that the tip of the outer tube 604 can be brought closer to the tip of the scissor blades compared to the tip of the inner tube 603. The outer tube 604 can be electrically isolated from the scissor blades 1201, 1202. This allows a cauterization with the tip of the outer tube 604 and the tip of at least one of the scissor blades 1201, 1202 as the two electric poles of a bipolar diathermy.

Although not shown in the drawing, the scissors can similarly combined with the configuration with the elongated element comprising two electrically conductive cores, as described with reference to Figs. 1 to 5. The scissor blades 1201 and 1202 can act as two bipolar diathermy electrodes. The two scissor blades 1201 and 1202 may be electrically isolated from each other by means of an electrically isolating layer. Such a layer may be fixed to at least one side of at least one of the scissor blades 1201, 1202, wherein said at least one side facing the other one of the scissor blades 1201, 1202.

Fig. 13A shows the scissor blades in an open position. Fig. 13B shows the scissor blades in a close position. By moving the tube 1203 with respect to the elongated element 1205, the scissor blades can be controlled to assume either of these positions, or an intermediate position.

It is noted that the embodiments with the scissors could also be manufactured in a larger size, with a tube diameter of more than 1 millimeter, to generate larger diathermy scissor instruments. Also the embodiments with forceps may be manufactured in larger size, with a tube diameter of more than 1 millimeter, to generate larger diathermy forceps instruments.

It is also possible to produce scissor and/or forceps instruments with more than one elongated element. For example, each forceps jaw or each scissor blade could be fixed to a different elongated element. For example, each elongated element could comprise an electrically conductive core.

To prevent tissue from sticking to the surgical instrument as a consequence of the cauterization, materials may be employed that do not stick. Such materials may be preferably applied for the forceps jaws, scissor blades, and/or the tip of the (outer) tube. Examples of suitable materials include noble metals. The relevant portions may be gilt or provided with a layer of gold or silver. Other suitable materials include rhodium and titanium. An advantage of titanium is that it can resist heat well. Therefore it may be advantageous, in certain embodiments, to use titanium for the forceps and/or for the distal end of the tube or tubes.

Throughout the drawings, the bipolar electrodes have been tentatively labeled "+" and "-". However, this is only illustrative. The polarity of the poles may be implemented differently.

The examples and embodiments described herein serve to illustrate rather than limit the invention. The person skilled in the art will be able to design alternative embodiments without departing from the scope of the claims. Reference signs placed in parentheses in the claims shall not be interpreted to limit the scope of the claims. Items described as separate entities in the claims or the description may be implemented as a single hardware or software item combining the features of the items described.

## Claims

1. A microsurgical fine gripping forceps for intraocular surgery, comprising:
a tube defining a conduit and having an outer diameter of at most 1 millimeter; and
forceps jaws protruding from the conduit at a distal end of the tube, the forceps jaws comprising at least a first forceps jaw and a second forceps jaw, wherein the forceps jaws are configured such as to allow grasping a vitreoretinal membrane;
**characterised in that** the forceps is a diathermy forceps, wherein at least two of the first forceps jaw, the second forceps jaw, and the tube are configured such as to form bipolar electrodes for providing a bipolar diathermy at the forceps jaws and/or a distal end of the tube.

2. The forceps of claim 1, further comprising an elongated element which extends through the conduit of the tube and is movable with respect to the tube and comprises at least one electrically conducting core which extends through the conduit of the tube, wherein the forceps jaws are fixed to the elongated element, and wherein the at least one electrically conducting core is electrically connected to at least one of the forceps jaws.

3. The forceps of claim 2,
wherein the elongated element comprises an electrically conducting first core, an electrically conducting second core, and an electrically isolating layer which electrically isolates the first core and the second core from one another;
wherein a first forceps jaw of the forceps jaws is electrically connected to the first core, and a second forceps jaw of the forceps jaws is electrically connected to the second core,
wherein the first core, the second core, and the isolating layer extend from the forceps jaws through the tube towards a proximal end of the tube.

4. The forceps of claim 3, wherein the isolating layer comprises an adhesive configured to fix the first core with respect to the second core.

5. The forceps of claim 3, wherein a cross section of the first core has a shape of a circular segment and a cross section of the second core has a shape of a circular segment, wherein a straight side of the cross section of the first core faces a straight side of the cross section of the second core.

6. The forceps of claim 3, comprising a further electrically isolating layer configured to electrically isolate the first core and/or the second core from the tube.

7. The forceps of claim 2, wherein the tube is electrically conducting, and wherein the forceps comprises an electrically isolating layer configured to electrically isolate the at least one core from the tube.

8. The forceps of claim 7, wherein the at least one core is configured to be electrically connected to a first pole of a source of electric energy and the tube is configured to be connected to a second pole of the source of electric energy.

9. The forceps of claim 7, wherein the tube is longitudinally displaceable with respect to the at least one core and the forceps jaws, so that the forceps jaws can be placed in a retracted position in which the forceps jaws are at least partially retracted inside the tube and an extended position in which the forceps jaws are extended further out of the tube than in the retracted position, and wherein the isolating layer is configured to electrically isolate the forceps jaws from the tube in the retracted position.

10. The forceps of claim 9, further comprising a further tube inside the tube, wherein the core extends through the further tube, wherein the forceps jaws are configured to pinch together when the further tube is moved in a distal direction and wherein the tube is movable in the distal direction so that the distal end of the tube can extend beyond the distal end of the further tube.

11. The forceps of claim 10, wherein the further electrically isolating layer is in between an inner surface of the tube and an outer surface of the further tube.

12. The forceps of claim 10, comprising a first control for controlling the movement of the further tube and a second control for controlling the movement of the tube.

13. The forceps of claim 1, further comprising a third control for controlling a switch to switch on and off an electric source.

14. A microsurgical cutting device for intraocular surgery, comprising:
a tube defining a conduit and having an outer diameter of at most 1 millimeter; and
a pair of scissor blades protruding from the conduit at a distal end of the tube, the scissor blades comprising at least a first scissor blade and a second scissor blade, wherein the scissor blades are configured such as to allow cutting a vitreoretinal membrane;
**characterised in that** the device is a diathermy device, wherein at least two of the first scissor blade, the second scissor blade, and the tube are configured such as to form bipolar electrodes for providing a bipolar diathermy at the scissor blades and/or a distal end of the tube.

15. The device of claim 14, further comprising an elongated element which extends through the conduit of the tube and is movable with respect to the tube and comprises at least one electrically conducting core which extends through the conduit of the tube, wherein the scissor blades are fixed to the elongated element, and wherein the at least one electrically conducting core is electrically connected to at least one of the scissor blades, wherein the tube is electrically conducting, and wherein the device comprises a further electrically isolating layer configured to electrically isolate the at least one core from the tube.

16. The device of claim 15, wherein the tube is longitudinally displaceable with respect to the at least one core and the scissor blades, so that the scissor blades can be placed in a retracted position in which the scissor blades are at least partially retracted inside the tube and an extended position in which the scissor blades are extended further out of the tube than in the retracted position, and wherein the isolating layer is configured to electrically isolate the scissor blades from the tube in the retracted position.

## Patentansprüche

1. Mikrochirurgische Feingreifzange für Augeninnenchirurgie, die folgendes aufweist:
ein Rohr, das eine Leitung definiert und einen Außendurchmesser von höchstens 1 Millimeter aufweist; und
Zangenbacken, die an einem distalen Ende des Rohrs aus der Leitung herausragen, wobei die Zangenbacken wenigstens eine erste Zangenbacke und eine zweite Zangenbacke aufweisen; wobei die Zangenbacken so konfiguriert sind, dass sie das Ergreifen einer vitreoretinalen Membran erlauben;
**dadurch gekennzeichnet, dass** die Zange eine Diathermiezange ist,
wobei wenigstens zwei der folgenden, die erste Zangenbacke, die zweite Zangenbacke und das Rohrs so konfiguriert sind, dass sie bipolare Elektroden zum Vorsehen einer bipolaren Diathermie an den Zangenbacken und/oder einem distalen Ende des Rohrs bilden.

2. Zange nach Anspruch 1, die ferner ein längliches Element aufweist, das sich durch die Leitung des Rohrs erstreckt und bezüglich des Rohrs bewegbar ist, und wenigstens einen elektrisch leitenden Kern aufweist, der sich durch die Leitung des Rohrs erstreckt, wobei die Zangenbacken an dem länglichen Element fixiert sind, und wobei der wenigstens eine elektrisch leitende Kern mit wenigstens einer der Zangenbacken elektrisch verbunden ist.

3. Zange nach Anspruch 2,
wobei das längliche Element einen elektrisch leitenden ersten Kern, einen elektrisch leitenden zweiten Kern und eine elektrische Isolierschicht aufweist, die den ersten Kern und den zweiten Kern elektrisch voneinander isoliert;
wobei eine erste Zangenbacke der Zangenbacken elektrisch mit dem ersten Kern verbunden ist und eine zweite Zangenbacke der Zangenbacken elektrisch mit dem zweiten Kern verbunden ist,
wobei der erste Kern, der zweite Kern und die Isolierschicht sich von den Zangenbacken durch das Rohr in Richtung eines proximalen Endes des Rohrs erstrecken.

4. Zange nach Anspruch 3, wobei die Isolierschicht einen Klebstoff aufweist, der konfiguriert ist, um den ersten Kern bezüglich des zweiten Kerns zu fixieren.

5. Zange nach Anspruch 3, wobei ein Querschnitt des ersten Kerns die Form eines Kreissegments hat und ein Querschnitt des zweiten Kerns die Form eines Kreissegments hat, wobei eine gerade Seite des Querschnitts des ersten Kerns zu einer geraden Seite des Querschnitts des zweiten Kerns hinweist.

6. Zange nach Anspruch 3, die eine weitere elektrische Isolierschicht aufweist, die konfiguriert ist, um den ersten Kern und/oder den zweiten Kern von dem Rohr elektrisch zu isolieren.

7. Zange nach Anspruch 2, wobei das Rohr elektrisch leitend ist und wobei die Zange eine elektrische Isolierschicht aufweist, die konfiguriert ist, um den wenigstens einen Kern von dem Rohr elektrisch zu isolieren.

8. Zange nach Anspruch 7, wobei der wenigstens eine Kern so konfiguriert ist, dass er elektrisch mit einem ersten Pol einer elektrischen Energiequelle verbindbar ist, und das Rohr so konfiguriert ist, dass es mit einem zweiten Pol der elektrischen Energiequelle verbindbar ist.

9. Zange nach Anspruch 7, wobei das Rohr bezüglich des wenigstens einen Kerns und der Zangenbacken in Längsrichtung verschiebbar ist, so dass die Zangenbacken in eine eingefahrene Position gebracht werden können, in der die Zangenbacken wenigstens teilweise in das Rohr eingefahren sind, und in eine ausgefahrene Position, in der die Zangenbacken weiter aus dem Rohr ausgefahren sind als in der eingefahrenen Position, und wobei die Isolierschicht konfiguriert ist, um die Zangenbacken in der eingefahrenen Position elektrisch vom Rohr zu isolieren.

10. Zange nach Anspruch 9, die ferner ein weiteres Rohr innerhalb des Rohrs aufweist, wobei sich der Kern durch das weitere Rohr erstreckt, wobei die Zangenbacken so konfiguriert sind, dass sie sich zusammendrücken, wenn das weitere Rohr in eine distale Richtung bewegt wird, und wobei das Rohr in die distale Richtung bewegbar ist, so dass sich das distale Ende des Rohrs über das distale Ende des weiteren Rohrs hinaus erstrecken kann.

11. Zange nach Anspruch 10, wobei sich die weitere elektrische Isolierschicht zwischen einer Innenfläche des Rohrs und einer Außenfläche des weiteren Rohrs befindet.

12. Zange nach Anspruch 10, die eine erste Steuerung zum Steuern der Bewegung des weiteren Rohrs und eine zweite Steuerung zum Steuern der Bewegung des Rohrs aufweist.

13. Zange nach Anspruch 1, die ferner eine dritte Steuerung zum Steuern eines Schalters zum Ein-und Ausschalten einer elektrischen Quelle aufweist.

14. Mikrochirurgische Schneidvorrichtung für Augeninnenchirurgie, die Folgendes aufweist:
ein Rohr, das eine Leitung definiert und einen Außendurchmesser von höchstens 1 Millimeter aufweist; und
ein Paar Scherenklingen, die an einem distalen Ende des Rohrs aus der Leitung herausragen, wobei die Scherenklingen wenigstens ein erstes Scherenklinge und eine zweite Scherenklinge aufweisen, wobei die Scherenklingen so konfiguriert sind, dass sie das Schneiden einer vitreoretinalen Membran erlauben;
**dadurch gekennzeichnet**, das die Vorrichtung eine Diathermievorrichtung ist,
wobei wenigstens zwei der folgenden, die erste Scherenklinge, die zweite Scherenklinge und das Rohr so konfiguriert sind, dass sie bipolare Elektroden zum Vorsehen einer bipolaren Diathermie an den Scherenklingen und/oder einem distalen Ende des Rohrs bilden.

15. Vorrichtung nach Anspruch 14, die ferner ein längliches Element aufweist, das sich durch die Leitung des Rohrs erstreckt und bezüglich des Rohrs bewegbar ist und die wenigstens einen elektrisch leitenden Kern aufweist, der sich durch die Leitung des Rohrs erstreckt, wobei die Scherenklingen an dem länglichen Element fixiert sind und wobei der wenigstens eine elektrisch leitende Kern mit wenigstens einem der Scherenklingen elektrisch verbunden ist, wobei das Rohr elektrisch leitend ist und wobei die Vorrichtung eine weitere elektrische Isolierschicht aufweist, die konfiguriert ist, um den wenigstens einen Kern elektrisch vom Rohr zu isolieren.

16. Vorrichtung nach Anspruch 15, wobei das Rohr bezüglich des wenigstens einen Kerns und der Scherenklingen in Längsrichtung verschiebbar ist, so dass die Scherenklingen in eine eingefahrene Position gebracht werden können, in der die Scherenklingen wenigstens teilweise in das Rohr eingefahren sind, und in eine ausgefahrene Position, in der die Scherenklingen weiter aus dem Rohr ausgefahren sind als in der eingefahrenen Position, und wobei die Isolierschicht konfiguriert ist, um die Scherenklingen in der eingefahrenen Position elektrisch vom Rohr zu isolieren.

## Revendications

1. Pince de préhension fine microchirurgicale pour chirurgie intraoculaire, comprenant :
un tube définissant un conduit et ayant un diamètre externe de 1 millimètre au plus ; et
des mâchoires de pince faisant saillie du conduit au niveau d'une extrémité distale du tube, les mâchoires de pince comprenant au moins une première mâchoire de pince et une seconde mâchoire de pince, dans laquelle les mâchoires de pince sont configurées de sorte à permettre la saisie d'une membrane vitréo-rétinienne ;
**caractérisée en ce que** la pince est une pince de diathermie,
dans laquelle au moins deux de la première mâchoire de pince, de la seconde mâchoire de pince et du tube sont configurés de sorte à former des électrodes bipolaires pour fournir une diathermie bipolaire au niveau des mâchoires de pince et/ou d'une extrémité distale du tube.

2. Pince selon la revendication 1, comprenant en outre un élément allongé qui s'étend à travers le conduit du tube et est mobile par rapport au tube et comprend au moins un noyau électroconducteur qui s'étend à travers le conduit du tube, dans laquelle les mâchoires de pince sont fixées à l'élément allongé, et dans laquelle l'au moins un noyau électroconducteur est connecté électriquement à au moins l'une des mâchoires de pince.

3. Pince selon la revendication 2,
dans laquelle l'élément allongé comprend un premier noyau électroconducteur, un second noyau électroconducteur, et une couche électriquement isolante qui isole électriquement le premier noyau et le second noyau l'un de l'autre ;
dans laquelle une première mâchoire de pince des mâchoires de pince est connectée électriquement au premier noyau, et une seconde mâchoire de pince des mâchoires de pince est connectée électriquement au second noyau,
dans laquelle le premier noyau, le second noyau et la couche isolante s'étendent depuis les mâchoires de pince à travers le tube vers une extrémité proximale du tube.

4. Pince selon la revendication 3, dans laquelle la couche isolante comprend un adhésif configuré pour fixer le premier noyau par rapport au second noyau.

5. Pince selon la revendication 3, dans laquelle une section transversale du premier noyau a la forme d'un segment circulaire et une section transversale du second noyau a la forme d'un segment circulaire, dans laquelle un côté rectiligne de la section transversale du premier noyau fait face à un côté rectiligne de la section transversale du second noyau.

6. Pince selon la revendication 3, comprenant une autre couche électriquement isolante configurée pour isoler électriquement le premier noyau et/ou le second noyau du tube.

7. Pince selon la revendication 2, dans laquelle le tube est électroconducteur, et dans laquelle la pince comprend une couche électriquement isolante configurée pour isoler électriquement l'au moins un noyau du tube.

8. Pince selon la revendication 7, dans laquelle l'au moins un noyau est configuré pour être connecté électriquement à un premier pôle d'une source d'énergie électrique et le tube est configuré pour être connecté à un second pôle de la source d'énergie électrique.

9. Pince selon la revendication 7, dans laquelle le tube peut être déplacé longitudinalement par rapport à l'au moins un noyau et aux mâchoires de pince, de telle sorte que les mâchoires de pince peuvent être placées dans une position rétractée dans laquelle les mâchoires de pince sont au moins partiellement rétractées à l'intérieur du tube et une position étendue dans laquelle les mâchoires de pince sont davantage étendues hors du tube que dans la position rétractée, et dans laquelle la couche isolante est configurée pour isoler électriquement les mâchoires de pince du tube dans la position rétractée.

10. Pince selon la revendication 9, comprenant en outre un autre tube à l'intérieur du tube, dans laquelle le noyau s'étend à travers l'autre tube, dans laquelle les mâchoires de pince sont configurées pour pincer ensemble lorsque l'autre tube est déplacé dans une direction distale et dans laquelle le tube est mobile dans la direction distale de telle sorte que l'extrémité distale du tube peut s'étendre au-delà de l'extrémité distale de l'autre tube.

11. Pince selon la revendication 10, dans laquelle l'autre couche électriquement isolante est intercalée entre une surface interne du tube et une surface externe de l'autre tube.

12. Pince selon la revendication 10, comprenant une première commande pour commander le mouvement de l'autre tube et une deuxième commande pour commander le mouvement du tube.

13. Pince selon la revendication 1, comprenant en outre une troisième commande pour commander à un interrupteur d'allumer et d'éteindre une source électrique.

14. Dispositif de coupe microchirurgical pour chirurgie intraoculaire, comprenant :
un tube définissant un conduit et ayant un diamètre externe d'1 millimètre au plus ; et
une paire de lames de ciseaux faisant saillie du conduit au niveau d'une extrémité distale du tube, les lames de ciseaux comprenant au moins une première lame de ciseaux et une seconde lame de ciseaux, dans lequel les lames de ciseaux sont configurées de sorte à permettre la coupe d'une membrane vitréo-rétinienne ;
**caractérisé en ce que** le dispositif est un dispositif de diathermie,
dans lequel au moins deux de la première lame de ciseaux, de la seconde lame de ciseaux et du tube sont configurés de sorte à former des électrodes bipolaires pour fournir une diathermie bipolaire au niveau des lames de ciseaux et/ou d'une extrémité distale du tube.

15. Dispositif selon la revendication 14, comprenant en outre un élément allongé qui s'étend à travers le conduit du tube et est mobile par rapport au tube et comprend au moins un noyau électroconducteur qui s'étend à travers le conduit du tube, dans lequel les lames de ciseau sont fixées à l'élément allongé, et dans lequel l'au moins un noyau électroconducteur est connecté électriquement à au moins l'une des lames de ciseaux, dans lequel le tube est électroconducteur, et dans lequel le dispositif comprend une autre couche électriquement isolante configurée pour isoler électriquement l'au moins un noyau du tube.

16. Dispositif selon la revendication 15, dans lequel le tube peut être déplacé longitudinalement par rapport à l'au moins noyau et aux lames de ciseaux, de telle sorte que les lames de ciseaux peuvent être placées dans une position rétractée dans laquelle les lames de ciseaux sont au moins partiellement rétractées à l'intérieur du tube et une position étendue dans laquelle les lames de ciseaux sont davantage étendues hors du tube que dans la position rétractée, et dans lequel la couche isolante est configurée pour isoler électriquement les lames de ciseaux du tube dans la position rétractée.
